(19) Europäisches Patentamt
European Patent Office
Office européen des brevets

(11) Veröffentlichungsnummer: **0 493 700 A1**

(12) **EUROPÄISCHE PATENTANMELDUNG**

(21) Anmeldenummer: **91120867.6**

(22) Anmeldetag: **05.12.91**

(51) Int. Cl.[5]: **C09B 57/00**, G11B 7/24, C07C 69/608, C07C 69/007, C07C 69/76, C07C 233/05, C07C 233/65, C07C 233/10

(30) Priorität: **20.12.90 DE 4040907**

(43) Veröffentlichungstag der Anmeldung: **08.07.92 Patentblatt 92/28**

(84) Benannte Vertragsstaaten: **CH DE FR GB IT LI**

(71) Anmelder: **BASF Aktiengesellschaft**
**Carl-Bosch-Strasse 38**
**W-6700 Ludwigshafen(DE)**

(72) Erfinder: **Schmitt, Michael, Dr.**
**Freudenbergstrasse 18**
**W-6940 Weinheim(DE)**
Erfinder: **Albert, Bernhard, Dr.**
**Rietburgstrasse 13**
**W-6701 Maxdorf(DE)**
Erfinder: **Brosius, Sibylle, Dr.**
**Cordovastrasse 29**
**W-6700 Ludwigshafen(DE)**
Erfinder: **Schomann, Klaus Dieter, Dr.**
**Kopernikusstrasse 47**
**W-6700 Ludwigshafen(DE)**
Erfinder: **Kuppelmaier, Harald, Dr.**
**In den Bannzaeuen 17**
**W-6701 Goennheim(DE)**

(54) **Verdoppelte Azulenquadratsäurefarbstoffe, deren Zwischenprodukte sowie optisches Aufzeichnungsmedium.**

(57) Verdoppelte Azulenquadratsäurefarbstoffe der Formel

EP 0 493 700 A1

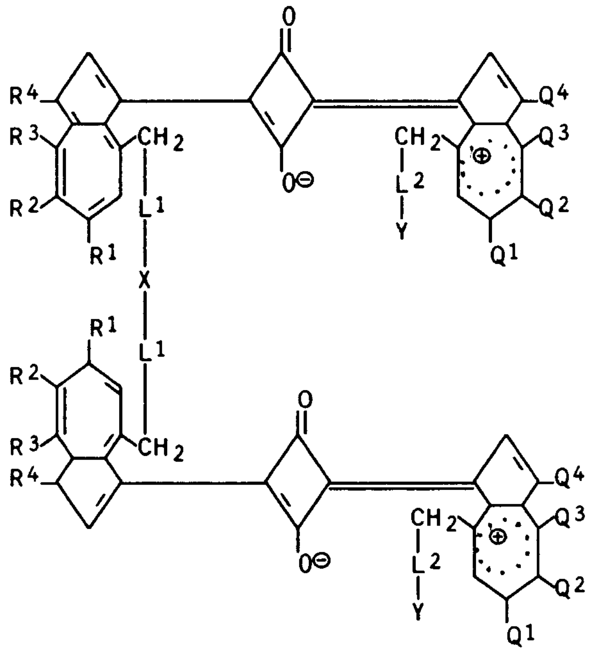

in der

| | |
|---|---|
| X | ein Brückenglied, |
| Y | Wasserstoff, Cyano oder einen Rest der Formel $CO\text{-}OR^5$, $CO\text{-}NHR^5$, $OR^5$, $O\text{-}CO\text{-}OR^5$, $NH\text{-}CO\text{-}R^5$, $NH\text{-}CO\text{-}OR^5$ oder $NH\text{-}SO_2\text{-}R^5$, worin $R^5$ für Wasserstoff, $C_1$-$C_{12}$-Alkyl, $C_2$-$C_{12}$-Alkenyl, $C_5$-$C_7$-Cycloalkyl oder gegebenenfalls substituiertes Phenyl steht, |
| $L^1$ und $L^2$ | unabhängig voneinander eine chemische Bindung oder gegebenenfalls substituiertes $C_1$-$C_{12}$-Alkylen und |
| $R^1$, $R^2$, $R^3$, $R^4$, $Q^1$, $Q^2$, $Q^3$ und $Q^4$ | unabhängig voneinander Wasserstoff oder gegebenenfalls substituiertes $C_1$-$C_{12}$-Alkyl |

bedeuten, deren Zwischenprodukte sowie ein optisches Aufzeichnungsmedium, enthaltend die neuen Farbstoffe.

Die vorliegende Erfindung betrifft neue verdoppelte Azulenquadratsäurefarbstoffe der Formel I

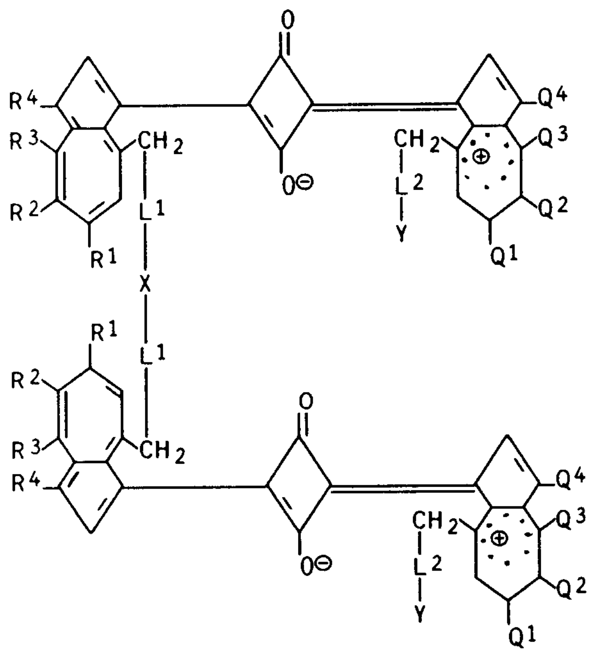

(I),

in der

X einen Rest der Formel CO-O-M-O-CO, CO-$NR^5$-M-$NR^5$-CO, O-CO-M-CO-O oder $NR^5$-CO-M-CO-$NR^5$, worin $R^5$ für Wasserstoff, $C_1$-$C_{12}$-Alkyl, $C_2$-$C_{12}$-Alkenyl, $C_5$-$C_7$-Cycloalkyl oder gegebenenfalls substituiertes Phenyl und M für $C_1$-$C_{12}$-Alkylen, Phenylen, Biphenylen oder einen Rest der Formel $(C_2H_4O\text{-})_nC_2H_4$ oder

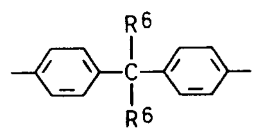

stehen, worin n die Bedeutung von 1 bis 10 und $R^6$ jeweils die Bedeutung von Wasserstoff oder $C_1$-$C_4$-Alkyl besitzen,

Y Wasserstoff, Cyano oder einen Rest der Formel CO-$OR^5$, CO-$NHR^5$, $OR^5$, O-CO-$OR^5$, NH-C0-$R^5$, NH-CO-$OR^5$ oder NH-$SO_2$-$R^5$, worin $R^5$ jeweils die obengenannte Bedeutung besitzt,

$L^1$ und $L^2$ unabhängig voneinander jeweils eine chemische Bindung oder $C_1$-$C_{12}$-Alkylen, das gegebenenfalls durch Phenyl substituiert ist, und

$R^1$, $R^2$, $R^3$, $R^4$, $Q^1$, $Q^2$, $Q^3$ und $Q^4$ unabhängig voneinander jeweils Wasserstoff oder $C_1$-$C_{12}$-Alkyl, das durch Halogen, $C_1$-$C_{12}$-Alkoxy, gegebenenfalls substituiertes Phenyl, $C_1$-$C_{12}$-Alkoxycarbonyl oder Cyano substituiert sein kann,

bedeuten, mit der Maßgabe, daß wenn $R^4$ und/oder $Q^4$ Wasserstoff bedeuten, an den Azulenringen die Ringpositionen der Substituenten $CH_2$-$L^1$-X-$L^1$-$CH_2$ und $R^3$ und/oder $CH_2$-$L^2$-Y und $Q^3$ auch gegeneinander vertauscht sein können, deren Zwischenprodukte sowie ein optisches Aufzeichnungsmedium, enthaltend die neuen Farbstoffe.

Zur kostengünstigen Herstellung optischer Datenaufzeichnungsträger werden Farbstoffe mit besonderen Eigenschaften benötigt. Diese Farbstoffe sollten

- eine starke Absorption zwischen 700 und 900 nm aufweisen, um mit Halbleiterlasern beschreibbare Schichten zu liefern,
- in Schicht eine hohe Reflektivität im nahen Infrarot (700 bis 900 nm) aufweisen, um mit einem einfachen Schichtaufbau (ohne Reflektorschicht) auszukommen,
- eine hohe Löslichkeit aufweisen, um beispielsweise die dünne Speicherschicht durch Spincoating auf

einen Träger aufbringen zu können und

- in dünnen Schichten eine hohe Stabilität aufweisen.

Aus der EP-A-310 080 und EP-A-341 541 sowie den älteren Patentanmeldungen EP-A-424777 und EP-A-427 007 sind bereits Azulenquadratsäurefarbstoffe bekannt.

Viele der bisher bekannten Speichermaterialien weisen jedoch häufig zumindest in einem der genannten Anforderungspunkte Mängel auf.

Aufgabe der vorliegenden Erfindung war es daher, neue Farbstoffe bereitzustellen, bei denen die obengenannten Mängel nicht mehr oder höchstens nur in äußerst geringem Maße auftreten.

Demgemäß wurden die oben näher bezeichneten verdoppelten Azulenquadratsäurefarbstoffe der Formel I gefunden.

Alle in der obengenannten Formel I auftretenden Alkyl-, Alkylen- und Alkenylgruppen können sowohl geradkettig als auch verzweigt sein.

Wenn in Formel I substituierte Phenylgruppen auftreten, können als Substituenten z. B. $C_1$-$C_4$-Alkyl, $C_1$-$C_4$-Alkoxy oder Halogen, in Betracht kommen.

Als Halogen ist jeweils Fluor, Chlor oder Brom bevorzugt.

Reste $L^1$ und $L^2$ sind z. B. Methylen, Ethylen, 1,2- oder 1,3-Propylen, 1,2-, 1,3-, 2,3- oder 1,4-Butylen, Pentamethylen, Hexamethylen, Heptamethylen, Octamethylen, Nonamethylen, Decamethylen, Undecamethylen, Dodecamethylen, Phenylethylen oder 1-Phenyl-1,3-propylen.

Reste $R^1$, $R^2$, $R^3$, $R^4$, $R^5$, $R^6$, $Q^1$, $Q^2$, $Q^3$ und $Q^4$ in Formel I sind beispielsweise Methyl, Ethyl, Propyl, Isopropyl, Butyl, Isobutyl, sec-Butyl oder tert-Butyl.

Reste $R^1$, $R^2$, $R^3$, $R^4$, $R^5$, $Q^1$, $Q^2$, $Q^3$ und $Q^4$ sind weiterhin z.B. Pentyl, Isopentyl, Neopentyl, tert-Pentyl, Hexyl, 2-Methylpentyl, Heptyl, Octyl, 2-Ethylhexyl, Nonyl, Decyl, Undecyl oder Dodecyl.

Reste $R^5$ sind weiterhin z.B. Vinyl, Allyl, Methallyl, Ethallyl, Cyclopentyl, Cyclohexyl, Cycloheptyl, 2- oder 4-Methylphenyl, 2- oder 4-Methoxyphenyl oder 2- oder 4-Chlorphenyl.

Reste $R^1$, $R^2$, $R^3$, $R^4$, $Q^1$, $Q^2$, $Q^3$ und $Q^4$ sind weiterhin z. B. Fluormethyl, Chlormethyl, Difluormethyl, Trifluormethyl, Trichlormethyl, 2-Fluorethyl, 2-Chlorethyl, 2-Bromethyl, 1,1,1-Trifluorethyl, Heptafluorpropyl, 4-Chlorbutyl, 5-Fluorpentyl, 6-Chlorhexyl, Cyanomethyl, 2-Cyanoethyl, 3-Cyanopropyl, 2-Cyanobutyl, 4-Cyanobutyl, 5-Cyanopentyl, 6-Cyanohexyl, 2-Methoxyethyl, 2-Ethoxyethyl, 2-Propoxyethyl, 2-Isopropoxyethyl, 2-Butoxyethyl, 2-Methoxypropyl, 2-Ethoxypropyl, 3-Ethoxypropyl, 4-Ethoxybutyl, 4-Isopropoxybutyl, 5-Ethoxypentyl, 6-Methoxyhexyl, Benzyl, 1-Phenylethyl, 2-Phenylethyl, 4-Chlorbenzyl, 4-Methoxybenzyl, 2-(4-Methylphenyl)ethyl, Methoxycarbonylmethyl, Ethoxycarbonylmethyl, 2-Methoxycarbonylethyl, 2-Ethoxycarbonylethyl, 3-Methoxycarbonylpropyl, 3-Ethoxycarbonylpropyl, 4-Methoxycarbonylbutyl, 4-Ethoxycarbonylbutyl, 5-Methoxycarbonylpentyl, 5-Ethoxycarbonylpentyl, 6-Methoxycarbonylhexyl oder 6-Ethoxycarbonylhexyl.

Bevorzugt sind verdoppelte Azulenquadratsäurefarbstoffe der Formel I, der $R^1$, $R^2$, $R^3$, $R^4$, $Q^1$, $Q^2$, $Q^3$ und $Q^4$ jeweils Wasserstoff oder $C_1$-$C_6$-Alkyl bedeuten.

Besonders bevorzugt sind verdoppelte Azulenquadratsäurefarbstoffe der Formel I, in der $R^1$, $R^2$, $R^3$ und $R^4$ jeweils Wasserstoff oder $C_1$-$C_6$-Alkyl, $Q^1$ und $Q^3$ jeweils Wasserstoff, $Q^2$ Isopropyl und $Q^4$ Methyl bedeuten.

Insbesondere bevorzugt sind verdoppelte Azulenquadratsäurefarbstoffe der Formel I, in der $R^1$, $R^3$, $Q^1$ und $Q^3$ jeweils Wasserstoff, $R^2$ und $Q^2$ jeweils Isopropyl und $R^4$ und $Q^4$ jeweils Methyl bedeuten.

Von besonderer Bedeutung sind verdoppelte Azulenquadratsäurefarbstoffe der Formel I, in der $L^2$ eine chemische Bindung und Y Wasserstoff bedeutet.

Die neuen Farbstoffe der Formel I werden aus verdoppelten Azulenderivaten der Formel II

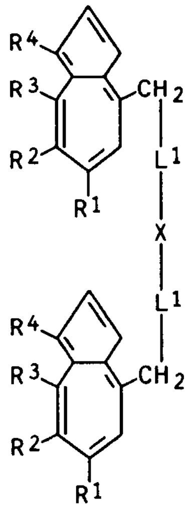

(II),

in der $L^1$, X, $R^1$, $R^2$, $R^3$ und $R^4$ jeweils die obengenannte Bedeutung besitzen, durch Umsetzung mit Quadratsäurederivaten der Formel III

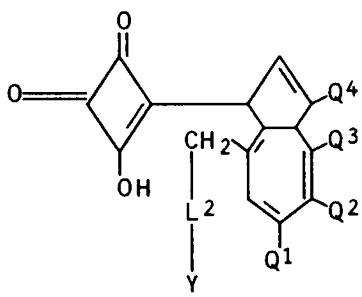

(III),

in der $L^2$, Y, $Q^1$, $Q^2$, $Q^3$ und $Q^4$ jeweils die obengenannte Bedeutung besitzen, im Molverhältnis 1:2 bis 1:3 erhalten.

Quadratsäurederivate der Formel III werden gemäß der folgenden Gleichung durch Reaktion der Azulenderivate IV mit Quadratsdäuredichlorid erhalten, wobei Y, $L^2$, $Q^1$, $Q^2$, $Q^3$ und $Q^4$ jeweils die obengenannte Bedeutung besitzen:

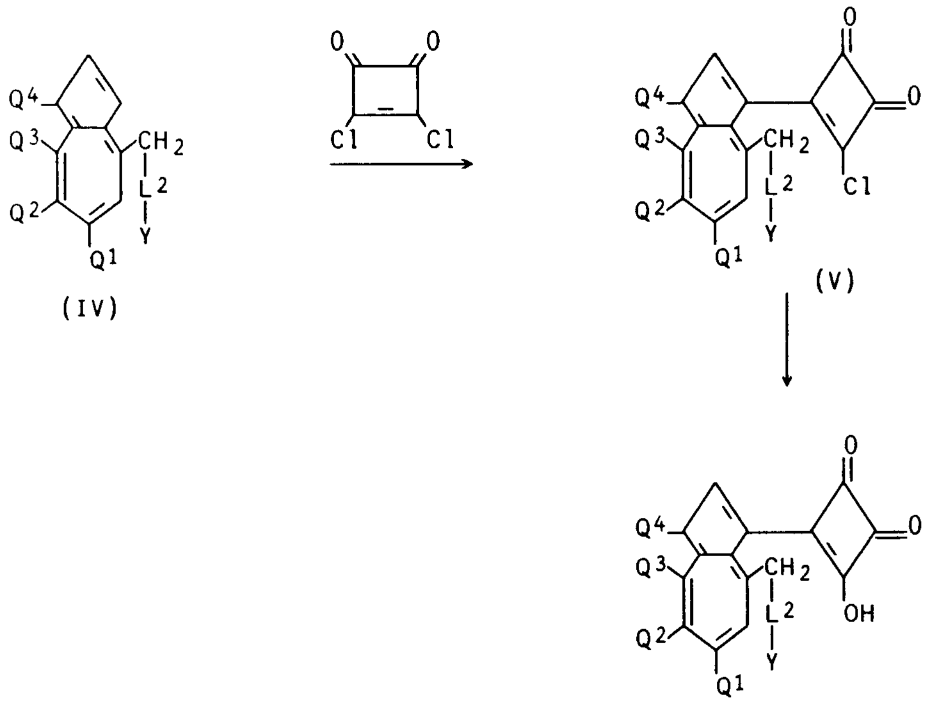

(IV)

(V)

(III)

Bei denjenigen Azulenderivaten der Formel II und IV, in denen $R^4$ und $Q^4$ Wasserstoff bedeuten, kann die Verknüpfung zur Quadratsäure an unterschiedlichen Ringpositionen des Fünfrings erfolgen, wobei isomere Produkte entstehen können, in denen die Ringpositionen der Substituenten $CH_2$-$L^1$-X-$L^1$-$CH_2$ und $R^3$ sowie $CH_2$-$L^2$-Y und $Q^3$, wie oben ausgeführt, gegeneinander vertauscht sind. Es sind nämlich dann Verbindungen, bei denen die Bindung zur Quadratsäure an derjenigen Seite erfolgt, an der der Substituent $CH_2$-$L^1$-X-$L^1$-$CH_2$ oder $CH_2$-$L^2$-Y gebunden ist, von solchen zu unterscheiden, bei denen die Bindung zur Quadratsäure an derjenigen Seite erfolgt, an der der Substituent $R^3$ oder $Q^3$ gebunden ist. Die isomeren Verbindungen können chromatographisch getrennt werden. Für die Anwendungen in Speicherschichten werden jedoch üblicherweise die Isomerengemische eingesetzt.

Die Erfindung betrifft weiterhin neue verdoppelte Azulenderivate der Formel II

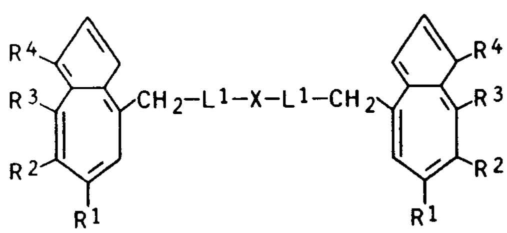

(II)

in der

X einen Rest der Formel CO-O-M-O-CO, CO-$NR^5$-M-$NR^5$-CO, O-CO-M-CO-O oder $NR^5$-CO-M-CO-$NR^5$, worin $R^5$ für Wasserstoff, $C_1$-$C_{12}$-Alkyl, $C_2$-$C_{12}$-Alkenyl, $C_5$-$C_7$-Cycloalkyl oder gegebenenfalls substituiertes Phenyl und M für $C_1$-$C_{12}$-Alkylen, Phenylen, Biphenylen oder einen Rest der Formel $(C_2H_4O\text{-})_nC_2H_4$ oder

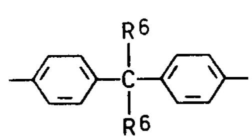

stehen, worin n die Bedeutung von 1 bis 10 und $R^6$ jeweils die Bedeutung von

Wasserstoff oder $C_1$-$C_4$-Alkyl besitzen,

$L^1$ eine chemische Bindung oder $C_1$-$C_{12}$-Alkylen, das gegebenenfalls durch Phenyl substituiert ist, und

$R^1$, $R^2$, $R^3$ und $R^4$ unabhängig voneinander jeweils Wasserstoff oder $C_1$-$C_{12}$-Alkyl, das durch Halogen, $C_1$-$C_{12}$-Alkoxy, gegebenenfalls substituiertes Phenyl, $C_1$-$C_{12}$-Alkoxycarbonyl oder Cyano substituiert sein kann, bedeuten.

Die verdoppelten Azulenderivate der Formel II erhält man beispielsweise:

- aus Azulenylalkancarbonsäuren, die beispielsweise die Formel VI

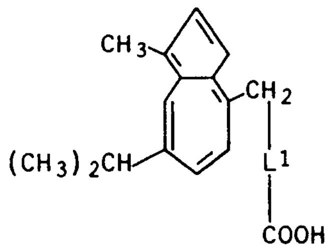

(VI)

aufweisen, in der $L^1$ die obengenannte Bedeutung besitzt, und bifunktionellen Hydroxyverbindungen oder bifunktionellen Aminen,

- aus Hydroxyalkylazulenen, die beispielsweise die Formel VII

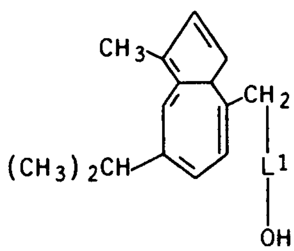

(VII)

aufweisen, in der $L^1$ die obengenannte Bedeutung besitzt, und bifunktionellen Säurechloriden,

- aus Azulenylalkylaminen, die beispielsweise die Formel VIII

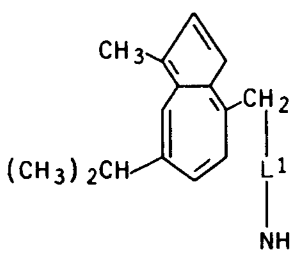

(VIII)

aufweisen, in der $L^1$ die obengenannte Bedeutung besitzt, und bifunktionellen Isocyanaten oder bifunktionellen Säurechloriden.

Die genannten Umsetzungen sind an sich bekannt und beispielsweise in Houben-Weyl "Methoden der Organischen Chemie" E5, Seiten 659 bis 684, Seiten 695 bis 700, Seiten 941 bis 964; E4, Seiten 181 bis 189, oder Seiten 352 bis 364, beschrieben.

Die Herstellung der Azulenderivate VI, VII und VIII erfolgt nach an sich bekannten Methoden und ist beispielsweise in der EP-A-310 080 oder der älteren deutschen Patentanmeldung P 40 39 437.9 beschrieben.

Eine weitere Aufgabe der vorliegenden Erfindung war es, ein neues optisches Aufzeichnungsmedium mit Azulenquadratsäurefarbstoffen als Speichermaterialien bereitzustellen, das in einfacher Weise hergestellt werden kann, das gut beschreibbar und anschließend auch gut lesbar ist, das eine hohe Stabilität der Speicherschichten aufweist und wobei das Signal:Rausch-Verhältnis möglichst hoch sein sollte.

Die Erfindung betrifft nun weiterhin ein optisches Aufzeichnungsmedium, enthaltend einen Träger sowie

einen strahlungsempfindlichen, Farbstoff und gegebenenfalls Bindemittel enthaltenden dünnen Beschichtungsfilm, wobei der Farbstoff die Formel I

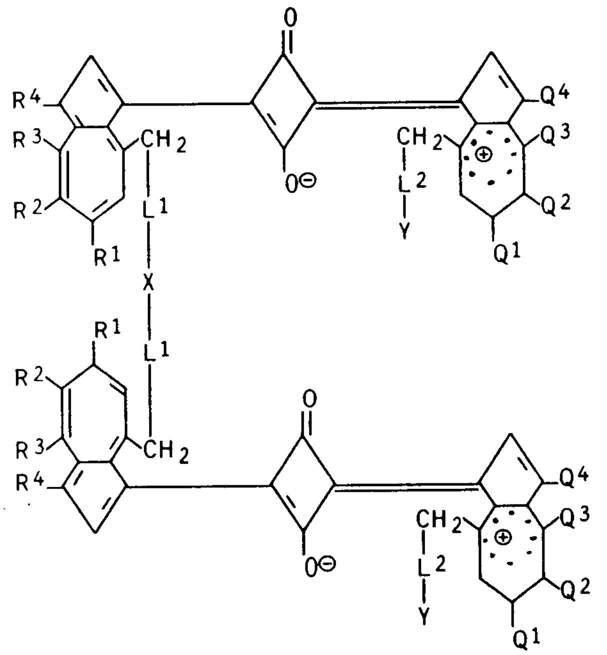

(I)

aufweist, in der

X einen Rest der Formel CO-O-M-O-CO, CO-$NR^5$-M-$NR^5$-CO, O-CO-M-CO-O oder $NR^5$-CO-M-CO-$NR^5$, worin $R^5$ für Wasserstoff, $C_1$-$C_{12}$-Alkyl, $C_2$-$C_{12}$-Alkenyl, $C_5$-$C_7$-Cycloalkyl oder gegebenenfalls substituiertes Phenyl und M für $C_1$-$C_{12}$-Alkylen, Phenylen, Biphenylen oder einen Rest der Formel $(C_2H_4O-)_nC_2H_4$ oder

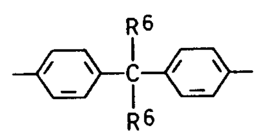

stehen, worin n die Bedeutung von 1 bis 10 und $R^6$ jeweils die Bedeutung von Wasserstoff oder $C_1$-$C_4$-Alkyl besitzen,

Y Wasserstoff, Cyano oder einen Rest der Formel CO-$OR^5$, CO-$NHR^5$, $OR^5$, O-CO-$OR^5$, NH-C0-$R^5$, NH-CO-$OR^5$ oder NH-$SO_2$-$R^5$, worin $R^5$ jeweils die obengenannte Bedeutung besitzt,

$L^1$ und $L^2$ unabhängig voneinander jeweils eine chemische Bindung oder $C_1$-$C_{12}$-Alkylen, das gegebenenfalls durch Phenyl substituiert ist, und

$R^1$, $R^2$, $R^3$, $R^4$, $Q^1$, $Q^2$, $Q^3$ und $Q^4$ unabhängig voneinander jeweils Wasserstoff oder $C_1$-$C_{12}$-Alkyl, das durch Halogen, $C_1$-$C_{12}$-Alkoxy, gegebenenfalls substituiertes Phenyl, $C_1$-$C_{12}$-Alkoxycarbonyl oder Cyano substituiert sein kann,

bedeuten, mit der Maßgabe, daß wenn $R^4$ und/oder $Q^4$ Wasserstoff bedeuten, an den Azulenringen die Ringpositionen der Substituenten $CH_2$-$L^1$-X-$L^1$-$CH_2$ und $R^3$ und/oder $CH_2$-$L^2$-Y und $Q^3$ auch gegeneinander vertauscht sein können.

Bevorzugt ist ein optisches Aufzeichnungsmedium, das verdoppelte Azulenquadratsäurefarbstoffe der Formel I enthält, in der $R^1$, $R^2$, $R^3$, $R^4$, $Q^1$, $Q^2$, $Q^3$ und $Q^4$ jeweils Wasserstoff oder $C_1$-$C_6$-Alkyl bedeuten.

Besonders bevorzugt ist ein optisches Aufzeichnungsmedium, das verdoppelte Azulenquadratsäurefarbstoffe der Formel I enthält, in der $R^1$, $R^2$, $R^3$ und $R^4$ jeweils Wasserstoff oder $C_1$-$C_6$-Alkyl, $Q^1$ und $Q^3$ jeweils Wasserstoff, $Q^2$ Isopropyl und $Q^4$ Methyl bedeuten.

Insbesondere bevorzugt ist ein optisches Aufzeichnungsmedium, das verdoppelte Azulenquadratsäurefarbstoffe der Formel I enthält, in der $R^1$, $R^3$, $Q^1$ und $Q^3$ jeweils Wasserstoff, $R^2$ und $Q^2$ jeweils Isopropyl

und $R^4$ und $Q^4$ jeweils Methyl bedeuten.

Als Träger kommen zweckmäßig transparente Träger, wie Glas oder Kunststoffe in Betracht. Geeignete Kunststoffe sind beispielsweise Poly(meth)acrylate, Polycarbonate, Polyester, Epoxide, Polyolefine, z.B. Polymethylpenten, Polyamid, Polyvinylchlorid, Polystyrol oder Polyvinylester.

Ein bevorzugtes Aufzeichnungsmedium weist einen Träger aus Polycarbonat oder Poly(meth)acrylaten, insbesondere aber Polycarbonat auf.

Weiterhin bevorzugt ist ein optisches Aufzeichnungsmedium, das 1 bis 30 Gew.-% bezogen auf Farbstoff, an Bindemittel enthält.

Die neuen verdoppelten Azulenquadratsäurefarbstoffe der Formel I zeigen gute optische Daten. Darüber hinaus sind bei den neuen Verbindungen die reinen Farbstoffschichten sehr stabil, d.h. sie weisen eine äußerst geringe Kristallisationsneigung auf. Bislang wurde nämlich keine Rekristallisation der reinen Farbstoffschicht beobachtet und somit kann auf den Zusatz polymerer Bindemittel verzichtet werden. Außerdem ist auch die Lichtechtheit (Stabilität) deutlich größer als bei bekannten Methinfarbstoffen, so daß der Zusatz von Stabilisatoren zur Schichtrezeptur auf ein Minimum begrenzt werden kann. Von besonderem Vorteil ist auch die gute Löslichkeit der neuen Farbstoffe I in den meisten organischen Lösungsmitteln, so daß diese Farbstoffe direkt (ohne Schutzschicht) auf strukturierte Kunststoffsubstrate, insbesondere Polycarbonatsubstrate, aufgeschleudert werden können.

Wie oben ausgeführt, enthält die aufzuschleudernde Lösung vorzugsweise ein Bindemittel, um eine gute Langzeitstabilität des Aufzeichnungsmediums zu gewährleisten und vor allem die optimale Viskosität der aufzuschleudernden Lösung einzustellen. Vorzugsweise enthält die Lösung dabei 1 bis 30 Gew.-%, bezogen auf den Gehalt an gelöstem Feststoff der aufzuschleudernden Lösung, eines Bindemittels. Als Bindemittel kommen z.B. Polyorganosiloxane, Epoxide, Poly(meth)acrylate, Polystyrolhomo- und copolymerisate, Polyvinylcarbazol, Polyvinylpyrrolidon, Polyimidazolcopolymere, Polyvinylestercopolymere, Polyvinylethercopolymere, Polyvinylidenchloridcopolymere, Acrylnitrilcopolymere, Polyvinylchlorid oder dessen Copolymere, Celluloseacetat oder Nitrocellulose in Betracht.

Ein bevorzugtes Aufzeichnungsmedium weist ein Bindemittel auf Basis eines Vinylpyrrolidon-Vinylacetat-Copolymeren oder eines Polyvinylchlorid-Polyvinylether-Copolymeren auf.

Das erfindungsgemäße optische Aufzeichnungsmedium wird zweckmäßig durch Aufschleudern einer Lösung, enthaltend organisches Lösungsmittel, Azulenquadratsäurefarbstoff I und gegebenenfalls Bindemittel, hergestellt. Zweckmäßig weist die aufzuschleudernde Lösung dabei einen Gehalt an gelöstem Feststoff von 1 bis 30 Gew.-%, bezogen auf die Lösung, auf.

Geeignete Lösungsmittel sind z.B. Propanol, Isopropanol, Butanol, Diacetonalkohol, Methylethylketon, Toluol, Bromoform, 1,1,2-Trichlorethan oder deren Mischungen.

Gegebenenfalls kann die Lösung noch 10 Gew.-%, bezogen auf den Gehalt an gelöstem Feststoff der aufzuschleudernden Lösung, an Additiven, z.B. Antioxidantien, Singulett-Sauerstoff-Quencher oder UV-Absorber, enthalten.

Bevorzugt enthält die aufzuschleudernde Lösung bis zu 5 Gew.-%, bezogen auf den Gehalt an gelöstem Feststoff der aufzuschleudernden Lösung, eine Mischung von mehreren Antioxidantien, Singulett-Sauerstoff-Quenchern und UV-Absorbern. Bei Anwendungen von solchen Antioxidantien, die ebenfalls im nahen Infrarot absorbieren, beispielsweise Nickelthiolenkomplexe, wie sie z. B. in der DE-A-3 505 750, DE-A-3 505 751 oder in S. H. Kim, M. Matsuoka, M. Yomoto, Y. Tsuchiva und T. Kitao, Dyes and Pigments, Band 8, S. 381 bis 388, 1987, beschrieben sind, können vorzugsweise bis zu 10 Gew.-%, bezogen auf den Gehalt an gelöstem Feststoff der aufzuschleudernden Lösung, in der Lösung enthalten sein.

Unter Aufschleudern ist dabei das Aufbringen der Lösung auf den in Rotation befindlichen Träger, der zweckmäßig eine runde Form aufweist, zu verstehen. Es ist aber auch möglich, die Lösung auf den zunächst ruhenden Träger aufzubringen und ihn anschließend in Rotation zu versetzen. Das Aufgeben auf den Träger erfolgt zweckmäßig mit einer Spritze oder Kapillaren oder mittels einer mechanischen Pumpe.

Die Rotation des Trägers erfolgt im allgemeinen mit einer Geschwindigkeit von 5 bis 7000 U/min, vorzugsweise 500 bis 5000 U/min, wobei das Aufschleudern der Lösung zweckmäßig bei geringerer Drehzahl (ca. 500 bis 2000 U/min) und das daran anschließende Trockenschleudern bei höherer Drehzahl (ca. 5000 bis 7000 U/min) vorgenommen wird.

Die Schichtdicke der gegenüber Laserlicht empfindlichen Schicht beträgt 40 bis 160 nm, vorzugweise 80 bis 120 nm. Sie ist abhängig von der Drehzahl, von der Konzentration und Viskosität der aufzuschleudernden Lösung sowie von der Temperatur.

Beim erfindungsgemäßen optischen Aufzeichnungsmedium liegt die gegenüber Laserlicht empfindliche Schicht in Form einer homogenen, dünnen, glatten Schicht vor, die eine hohe optische Qualität aufweist. So liegen die Reflektivitätswerte im allgemeinen in einem Bereich der größer als 12 % ist.

Das neue Aufzeichnungsmedium ist ferner bei der Wellenlänge der verwendeten Laserlichtquelle

hinreichend empfindlich, d. h. bei Einstrahlung von Lichtpulsen von wenigen nJ Energiegehalt, die auf Brennpunktdurchmesser von 1 ≦µm fokussiert sind, bilden sich Pits aus, wobei ein ausgezeichnetes Signal:Rausch-Verhalten erzielt wird.

Als Laserlichtquelle eignen sich wegen der geringen Größe des Bauelementes, des geringen Energiebedarfs und der Möglichkeit der direkten Modulation der optischen Ausgangsleistung durch Modulation des elektrischen Antriebsstromes Festkörper-Injektionslaser, die im nahen Infrarot emittieren, vor allem der AlGaAs-Laser, der im Wellenlängenbereich zwischen etwa 750 und 900 nm arbeitet, besonders gut.

Die folgenden Beispiele sollen die Erfindung näher erläutern.

I. Herstellung von verdoppelten Azulenderivaten der Formel

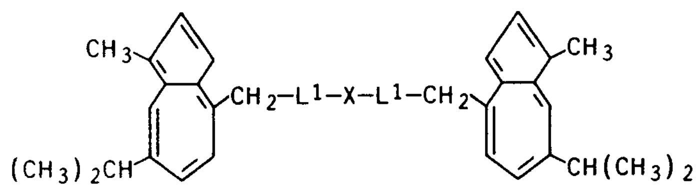

Beispiel 1

$L^1$-X-$L^1$: $C_2H_4$-O-CO-NH-$C_6H_{12}$-NH-CO-O-$C_2H_4$

21,8 g (0,09 mol) 7-Isopropyl-1-methyl-4-(3-hydroxypropyl)azulen wurden in 50 ml 1,1,1-Trichlorethan gelöst und mit 6,7 g (0,04 mol) Hexamethylendiisocyanat versetzt. Nach Zugabe von 1 Tropfen Didodecyldibutylstannan wurde 2 Stunden zum Rückfluß erhitzt. Nach beendeter Reaktion wurde das Lösungsmittel unter vermindertem Druck entfernt und der Rückstand an Kieselgel chromatographiert (Aceton/Methylenchlorid 1 : 9 v/v).

Man erhielt 25 g (96 %) verdoppeltes Azulenderivat als dunkelblauen Feststoff vom Schmp. 106 - 110 °C.

IR (Film): $\bar{\nu}$ = 3350 (-NH) ; 2957, 2932, 2865 (C-H); 1689 (C=O); 1526, 1463, 1438, 1388, 1255 $cm^{-1}$.

$^1$H-NMR ($CDCl_3$): $\delta$ = 1.2 - 1.5 (m, 8H); 1.3 (2s, 12H); 2.1 (q, 4H); 2.65 (s, 6H); 3.08 (mc, 2H); 3.15 (m, 8H); 4.15 (t, 4H); 4.7 (bs, 2H; NH); 6.95 (d, 2H); 7.25 (d, 2H); 7.35 (d, 2H), 7.60 (d, 2H), 8.15 (s, 2H) ppm.

$^{13}$C-NMR($CDCl_3$): $\delta$ = 12.83 (q, 2C) ; 24.70 (q, 4C); 26.37 (2C); 30.05 (2C); 30.71 (2C); 34.55 (2C); 38.25 (2C); 41.07 (2C); 64.66 (2C); 112.34 (2C); 124.43 (2C); 125.36 (2C); 133.18 (2C); 135.08 (2C); 136.41 (2C); 136.53 (2C); 137.33 (2C); 139.95 (2C); 147.82 (2C); 156.76 (2C) ppm.

MS (70eV): m/e = 653 (100 %, $C_{42}H_{56}N_2O_4^+$).

Beispiel 2

$L^1$-X-$L^1$: 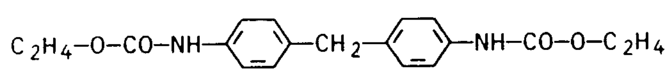

12,1 g (0,05 mol) 7-Isopropyl-1-methyl-4-(3-hydroxypropyl)azulen wurden in 50 ml 1,1,1-Trichlorethan gelöst und mit 5,0 g (0,02 mol) Diphenylmethan-4,4'-diisocyanat versetzt. Nach Zugabe von 1 Tropfen Didodecyldibutylstannan wurde 2 Stunden unter Rückfluß erhitzt. Nach beendeter Reaktion wurde das Lösungsmittel unter vermindertem Druck entfernt und der Rückstand an Kieselgel chromatographiert (Aceton/Methylenchlorid 1 : 40 v/v).

Man erhielt 8,4 g (57 %) verdoppeltes Azulenderivat als dunkelblauen Feststoff vom Schmp. 144 - 145 °C.

IR (KBr): $\bar{\nu}$ = 3440, 3360 (NH); 2957 (C-H); 1726, 1700 (C=O); 1600, 1521, 1412, 1309, 1227, 1204 $cm^{-1}$.

Beispiel 3

$L^1$-X-$L^1$: $C_2H_4$-O-CO-$C_4H_8$-CO-O-$C_2H_4$

12,1 g (0,05 mol) 7-Isopropyl-1-methyl-4-(3-hydroxypropyl)azulen wurden in 70 ml Dichlormethan gelöst. Nach Zugabe von 7,9 g (0,1 mol) Pyridin wurde bei Raumtemperatur eine Lösung von 4,6 g (0,025 mol) Adipinsäuredichlorid in 20 ml Dichlormethan gelöst zugetropft. Nach beendeter Reaktion wurde mit 200 ml Wasser hydrolisiert und mit 2N Salzsäure angesäuert. Die organische Phase wurde abgetrennt und über Natriumsulfat getrocknet. Nach Entfernen des Lösungsmittels unter vermindertem Druck wurde der Rückstand an Kieselgel chromatographiert (Petrolether/Ethylacetat 9 : 1 v/v).

Man erhielt 12,2 g (41 %) verdoppeltes Azulenderivat als dunkelblaues Öl.

Physikalische Daten:

IR (Film): $\bar{\nu}$ = 2958, 2868 (C-H); 1734 (C=O); 1462, 1388 $cm^{-1}$.

Beispiel 4

$L^1$-X-$L^1$: $CH(C_2H_5)-CH_2-O-CO-C_4H_8-CO-O-CH_2-CH(C_2H_5)$

13,5 g (0,05 mol) 3-(7-Isopropyl-1-methylazulen-4-yl)-2-ethylpropanolwurden analog Beispiel 3 mit Adipinsäuredichlorid zur Reaktion gebracht und aufgearbeitet.

Man erhielt 13,6 g (33 %) verdoppeltes Azulenderivat als dunkelblaues Öl.

Physikalische Daten:

IR (Film): $\bar{\nu}$ = 2959, 2932, 2872 (C-H); 1735 (C=O); 1461, 1388 $cm^{-1}$.

Beispiel 5

$L^1$-X-$L^1$: $CH_2-CO-O-C_2H_4-O-CO-CH_2$

Zu einer Lösung von 12,8 g (0,05 mol) 3-(7-Isopropyl-1-methylazulen-4-yl)-propionsäure und 1,6 g (0,025 mol) Ethylenglykol in 100 ml Dichlormethan wurden 11,8 g (0,06 mol) Dicyclohexylcarbodiimid in 10 ml Dichlormethan gelöst, bei 0°C zugetropft. Nach Zugabe von 0,5 g 4-(N,N-Dimethylamino)-pyridin wurde das Gemisch 4 Stunden bei 0°C gerührt. Vom Niederschlag (Dicyclohexylharnstoff) wurde abfiltriert, das Filtrat mehrmals mit 2N Salzsäure gewaschen und anschließend über Natriumsulfat getrocknet. Der nach Abziehen des Lösungsmittels verbleibende Rückstand wurde an Kieselgel (Petrolether/Ethylacetat 7 : 3 v/v) chromatographiert.

Man erhielt 9,8 g (73 %) verdoppeltes Azulenderivat als tiefblaues Öl.

Physikalische Daten:

IR (Film): $\bar{\nu}$ = 2957, 2925 (C-H); 1738 (C=O); 1555, 1461, 1387, 1291, 1150 $cm^{-1}$.

Beispiel 6

$L^1$-X-$L^1$: $CH_2-CO-O-C_2H_4-O-C_2H_4-O-CO-CH_2$

12,8 g (0,05 mol) 3-(7-Isopropyl-1-methylazulen-4-yl)propionsäure und 2,5 g Diethylenglykol wurden analog Beispiel 5 zur Reaktion gebracht und aufgearbeitet.

Man erhielt 11,4 g (78 %) verdoppeltes Azulenderivat als tiefblaues Öl.

Physikalische Daten:

IR (Film): $\bar{\nu}$ = 2958, 2927 (C-H); 1735 (C=O); 1461, 1387, 1293, 1258, 1177, 1136 $cm^{-1}$.

Beispiel 7

$L^1$-X-$L^1$: $CH_2-CO-O-C_2H_4-O-C_2H_4-O-C_2H_4-O-CO-CH_2$

12,8 g (0,05 mol) 3-(7-Isopropyl-1-methylazulen-4-yl)propionsäure und 3,5 g Triethylenglykol wurden

analog Beispiel 5 zur Reaktion gebracht und aufgearbeitet.

Man erhielt 11,2 g (72 %) verdoppeltes Azulenderivat als tiefblaues Öl.

Physikalische Daten:

IR (Film): $\bar{\nu}$ = 2957, 2926, 2867 (C-H); 1734 (C=O); 1461, 1387, 1293, 1177, 1145 $cm^{-1}$.

Beispiel 8

$L^1$-X-$L^1$: 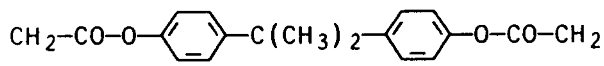

12,8 g (0,05 mol) 3-(7-Isopropyl-1-methylazulen-4-yl)propionsäure und 5,7 g (0,025 mol) 2,2-Bis(4-hydroxyphenyl)propan wurden analog Beispiel 5 zur Reaktion gebracht und aufgearbeitet.

Man erhielt 12,8 g (73 %) verdoppeltes Azulenderivat als tiefblaues Öl.

Physikalische Daten:

IR (Film): $\bar{\nu}$ = 2958, 2929 (C-H); 1758 (C=O); 1196, 1163, 1135 $cm^{-1}$.

Beispiel 9

$L^1$-X-$L^1$: $C_3H_6$-NH-CO-$C_6H_{12}$-CO-NH-$C_3H_6$

12,75 g (0,05 mol) 4-(7-Isopropyl-1-methyl-azulen-4-yl)butylamin wurden in 100 ml Dichlormethan gelöst und mit 7,9 g (0,1 mol) Pyridin versetzt.

Hierzu wurde eine Lösung von 5,3 g (0,025 mol) Hexan-1,6-dicarbonsäuredichloridin 10 ml Dichlormethan langsam zugetropft und 2 Stunden bei Raumtemperatur gerührt. Danach wurde der Ansatz mit 200 g Eiswasser hydrolisiert und mit 2N Salzsäure angesäuert. Die organische Phase wurde abgetrennt, über Natriumsulfat getrocknet und der nach Abziehen des Lösungsmittels verbleibende Rückstand an Kieselgel chromatographiert.

Man erhielt 15 g (91 %) verdoppeltes Azulenderivat als tiefblaues Öl.

Physikalische Daten:

IR (Film): $\bar{\nu}$ = 3300 (N-H); 1642, 1554 (-CO-NH-); 1463; 1387 $cm^{-1}$.

II. Herstellung von verdoppelten Azulenquadratsäurefarbstoffen

Beispiel 10

a) Zu einer Lösung aus 45 g (0,3 mol) Quadratsäuredichlorid in 250 ml Tetrahydrofuran wurden 59,5 g (0,3 mol) Guajazulen in 100 ml Tetrahydrofuran gelöst, bei Raumtemperatur langsam zugetropft. Nach beendeter Reaktion wurde die Lösung eingeengt und der Rückstand in 200 ml warmem Ethylacetat aufgenommen. Petrolether (Sdp. 40 bis 70 °C) wurde bis zur beginnenden Kristallisation zugegeben. Nach Abkühlen wurde der Niederschlag abgesaugt und mit Petrolether gewaschen. Man erhielt 73 g (78 %) 3-Chlor-4-(7-isopropyl-1,4-dimethylazulen-3-yl)cyclobut-3-en-1,2-dion als dunkelbraune Kristalle vom Schmp. 138 bis 139 °C.

Physikalische Daten:

IR (KBr): 3960, 3840 (C-H);, 1790, 1776, 1756 (C=0); 1531, 1508, 1396, 1366, 1229, 1049 $cm^{-1}$.

$^1$H-NMR ($CDCl_3$): $\delta$ = 1.40 (2s, 6H), 2.58 (s, 3H) 2.92 (s, 3H), 3.18 (m, 1H), 7.45 (d, 1H), 7.65 (d, 1H), 8.05 (s, 1H), 8.26 (s, 1H) ppm.

$^{13}$C-NMR ($CDCl_3$): $\delta$ = 12.76, 24.38 (2C), 27.43, 38.27, 112.29, 127.90, 133.52, 134.78, 137.41, 137.65, 140.25, 143.93, 148.34, 149.60, 173.43, 188.28, 190.74, 195.93 ppm.

MS: m/e = 312.5 ($C_{19}H_{17}Cl0_2^+$).

62,4 g (0,2 mol) 3-Chlor-4-(7-isopropyl-1,4-dimethylazulen-3-yl)cyclobut-3-en-1,2-dion wurden in 200 ml

Dioxan, 40 ml Wasser und 2 ml konz. Salzsäure 8 Stunden unter Rückfluß erhitzt. Das Lösungsmittel wurde unter vermindertem Druck entfernt und der verbleibende ölige Rückstand durch Zugabe von Dichlormethan kristallisiert. Man erhielt 36,5 g (62 %) 3-Hydroxy-4-(7-isopropyl-1,4-dimethylazulen-3-yl)-cyclobut-3-en-1,2-dion als dunkelbraune Kristalle vom Schmp. 120 bis 123 ° C. Die Substanz wurde ohne weitere Reinigung zur Farbstoffsynthese verwendet.

b) In analoger Weise wurde die Verbindung der Formel

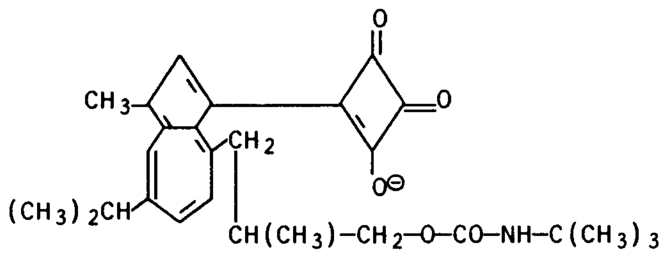

erhalten. Schmp. > 240 ° C (Zers.)

c) Eine Mischung aus 6,52 g (0,01 mol) verdoppeltem Azulenderivat aus Beispiel 1, 8,0 g (0,027 mol) 3-Hydroxy-4-(7-isopropyl-1,4-dimethylazulen-3-yl)cyclobut-3-en-1,2-dion, 40 ml Toluol und 40 ml n-Butanol wurde am Wasserabscheider 4 Stunden unter Rückfluß erhitzt. Nach Abziehen des Lösungsmittels unter vermindertem Druck wurde der Rückstand an Kieselgel (Methylenchlorid/Ethanol 9 : 1 v/v) chromatographiert.

Man erhielt 3,6 g (30 %) des verdoppelten Quadratsäurefarbstoffs der Formel

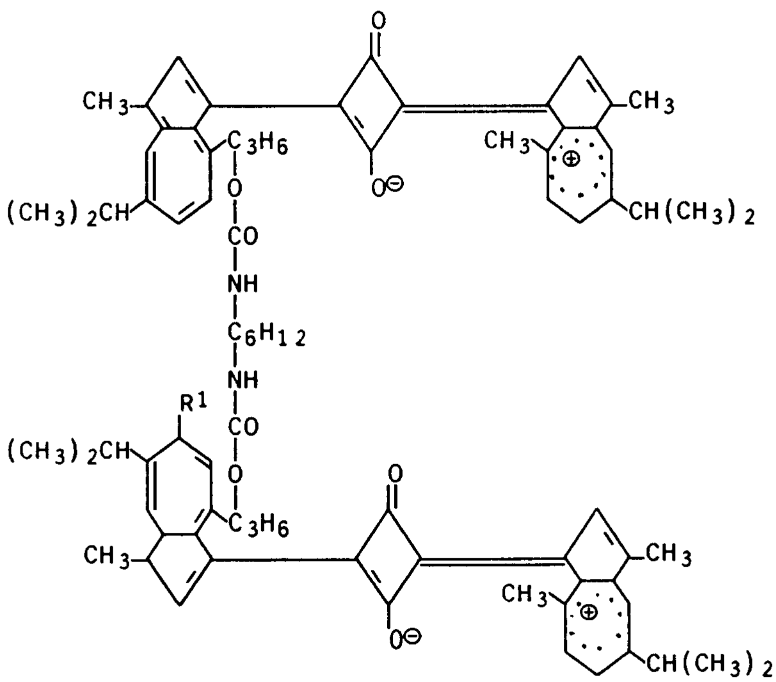

als tiefblaues Öl, das beim Anreiben mit Ethylacetat/Petrolether als blaugrüner Feststoff vom Schmp. 131 - 136 ° C kristallisierte.

Physikalische Daten:

IR (KBr): $\bar{\nu}$ = 2960, 2930, 2870 (C-H) ; 1714 (C = O); 1610, 1540, 1431, 1385, 1337, 1249, 1010 $cm^{-1}$.
UV ($CH_2Cl_2$): $\lambda_{max}(\epsilon)$ = 769(180 000) nm.

Analog Beispiel 10 wurden die in der folgenden Tabelle aufgeführten Quadratsäurefarbstoffe der Formel

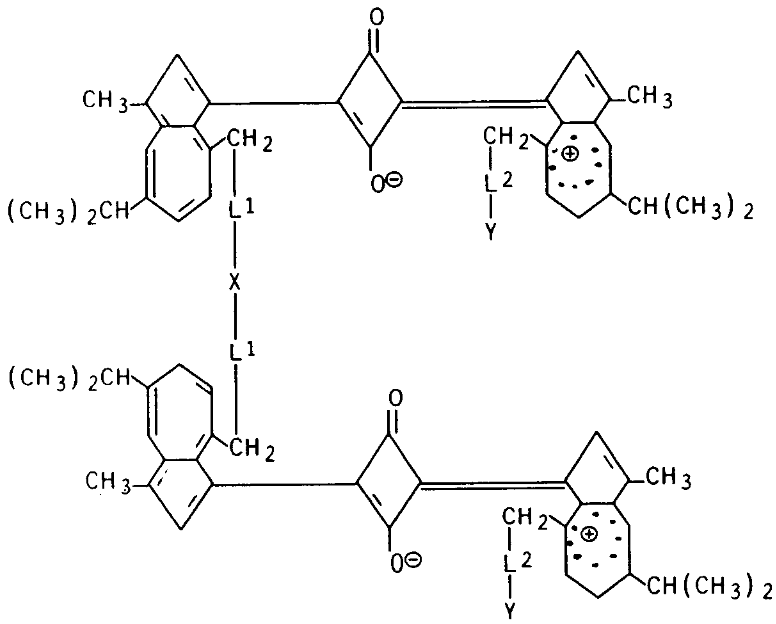

synthetisiert.

Tabelle

| Beispiel Nr. | $L^1-X-L^1$ | $L^2-Y$ | $\lambda_{max}$ [nm] ε in $CH_2Cl_2$ | Schm. [°C] |
|---|---|---|---|---|
| 11 | $C_2H_4-O-CO-NH-C_6H_{12}-NH-CO-O-C_2H_4$ | $CH(CH_3)-CH_2-O-CO-NH-C(CH_3)_3$ | 769 (170 000) | 136-141 |
| 12 | $C_2H_4-O-CO-NH-$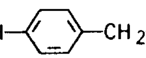$-CH_2-$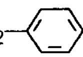$-NH-CO-O-C_2H_4$ | H | 772 (204 000) | 155-156 |
| 13 | $C_2H_4-O-CO-C_4H_8-CO-O-C_2H_4$ | H | 769 (195 000) | 110-120 |
| 14 | $CH(C_2H_5)-CH_2-O-CO-C_4H_8-CO-O-CH_2-CH(C_2H_5)$ | H | 770 (195 000) | 221-223 |
| 15 | $CH_2-CO-O-C_2H_4-O-CO-CH_2$ | H | 765 (185 000) | 93- 94 |
| 16 | $CH_2-CO-(OC_2H_4-)_2O-CO-CH_2$ | H | 764 (210 000) | 120-127 |
| 17 | $CH_2-CO-(OC_2H_4-)_3O-CO-CH_2$ | H | 764 (215 000) | 107-109 |
| 18 | $CH_2-CO-O-$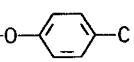$-C(CH_3)_2-$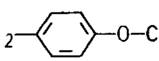$-O-CO-CH_2$ | H | 761 (220 000) | 102-103 |
| 19 | $C_3H_6-NH-CO-C_6H_{12}-CO-NH-C_3H_6$ | H | 765 (170 000) | 136-145 |

Beispiel 20

Eine 5 gew.%ige Lösung des Farbstoffs aus Beispiel 10 in Toluol wurde mit einer Spritze bei 2000 U/min auf eine rotierende Polymethylmethacrylatscheibe aufgetragen und dann das restliche Lösungsmittel

bei 5000 U/min abgeschleudert. Man erhielt eine homogene, hochreflektierende Farbstoffschicht, die sich mit einem Halbleiterlaser (λ = 830 nm) sehr gut beschreiben ließ. Die Informationen können mit gutem Kontrast wieder ausgelesen werden.

Beispiel 21

Eine 3 gew.%ige Lösung des Farbstoffs aus Beispiel 10, die 30 Gew.-%, bezogen auf den Gehalt an gelöstem Feststoff der Lösung, Polymethylmethacrylat enthielt, wurde analog Beispiel 20 auf eine gerillte Polycarbonatscheibe aufgeschleudert. Man erhielt eine homogene, hochreflektierende Farbstoffschicht, die auf dem Substrat gut haftet, die Spurrillen des Substrats gut abbildet und mit einem Halbleiterlaser (λ = 830 nm) sehr gut beschreibbar war. Die eingeschriebene Information war im Klimatest stabil und kann mit gutem Kontrast beliebig oft wieder ausgelesen werden.

Beispiel 22

Eine 2 gew.%ige Lösung des Farbstoffs aus Beispiel 10 in Propanol/Diacetonalkohol (1 : 1 v/v), die, bezogen auf den Gehalt an gelöstem Feststoff der Lösung, 10 Gew.-% eines Phenolharzes als Bindemittel und 5 Gew.-% 4-Octyl-4'-fluordiphenyl-dithiolennickel als Stabilisator enthielt, wurde analog Beispiel 20 auf eine gerillte Polycarbonatscheibe aufgeschleudert. Die erhaltene Speicherschicht war derjenigen aus Beispiel 20 in allen Belangen vergleichbar, wies aber eine erhöhte Stabilität gegenüber UV-Licht auf.

Beispiel 23

Eine 2 gew.%ige Lösung des Farbstoffs aus Beispiel 10 in Toluol, die, bezogen auf den Gehalt an gelöstem Feststoff der Lösung, 10 Gew.-% Polymethylmethacrylat und 5 Gew.-% Biscampheratodithiolennickel enthielt, wurde analog Beispiel 20 auf eine Glasscheibe aufgeschleudert. Die erhaltene Farbstoffschicht war homogen und zeigte eine hohe Grundreflektivität. Sie konnte mit einem Halbleiterlaser (λ = 780 nm) gut beschrieben werden. Die eingeschriebenen Informationen sind unter den üblichen Testbedingungen stabil und können beliebig oft wieder ausgelesen werden.

**Patentansprüche**

**1.** Verdoppelte Azulenquadratsäurefarbstoffe der Formel I

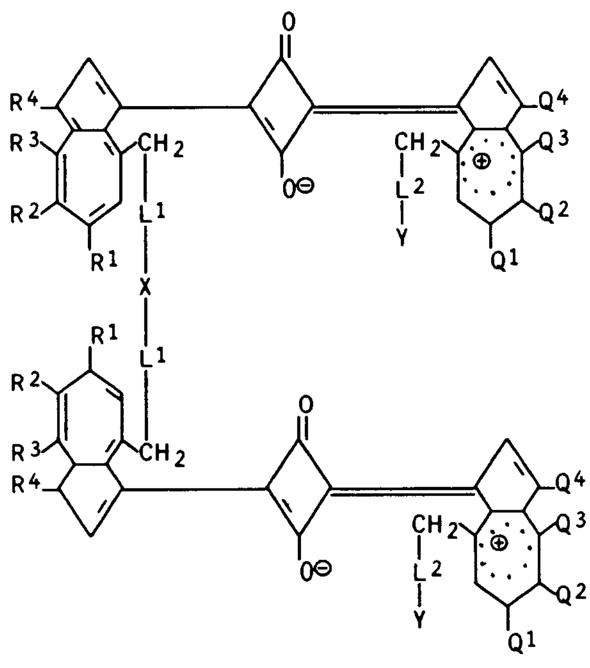

(I),

in der

X einen Rest der Formel CO-O-M-O-CO, CO-$NR^5$-M-$NR^5$-CO, O-

CO-M-CO-O oder $NR^5$-CO-M-CO-$NR^5$, worin $R^5$ für Wasserstoff, $C_1$-$C_{12}$-Alkyl, $C_2$-$C_{12}$-Alkenyl, $C_5$-$C_7$-Cycloalkyl oder gegebenenfalls substituiertes Phenyl und M für $C_1$-$C_{12}$-Alkylen, Phenylen, Biphenylen oder einen Rest der Formel $(C_2H_4O-)_nC_2H_4$ oder

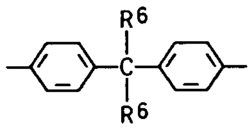

stehen, worin n die Bedeutung von 1 bis 10 und $R^6$ jeweils die Bedeutung von Wasserstoff oder $C_1$-$C_4$-Alkyl besitzen,

Y — Wasserstoff, Cyano oder einen Rest der Formel CO-$OR^5$, CO-$NHR^5$, $OR^5$, O-CO-$OR^5$, NH-C0-$R^5$, NH-CO-$OR^5$ oder NH-$SO_2$-$R^5$, worin $R^5$ jeweils die obengenannte Bedeutung besitzt,

$L^1$ und $L^2$ — unabhängig voneinander jeweils eine chemische Bindung oder $C_1$-$C_{12}$-Alkylen, das gegebenenfalls durch Phenyl substituiert ist, und

$R^1$, $R^2$, $R^3$, $R^4$, $Q^1$, $Q^2$, $Q^3$ und $Q^4$ — unabhängig voneinander jeweils Wasserstoff oder $C_1$-$C_{12}$-Alkyl, das durch Halogen, $C_1$-$C_{12}$-Alkoxy, gegebenenfalls substituiertes Phenyl, $C_1$-$C_{12}$-Alkoxycarbonyl oder Cyano substituiert sein kann,

bedeuten, mit der Maßgabe, daß wenn $R^4$ und/oder $Q^4$ Wasserstoff bedeuten, an den Azulenringen die Ringpositionen der Substituenten $CH_2$-$L^1$-X-$L^1$-$CH_2$ und $R^3$ und/oder $CH_2$-$L^2$-Y und $Q^3$ auch gegeneinander vertauscht sein können.

**2.** Verdoppelte Azulenquadratsäurefarbstoffe nach Anspruch 1, dadurch gekennzeichnet, daß $R^1$, $R^2$, $R^3$, $R^4$, $Q^1$, $Q^2$, $Q^3$ und $Q^4$ jeweils Wasserstoff oder $C_1$-$C_6$-Alkyl bedeuten.

**3.** Verdoppelte Azulenquadratsäurefarbstoffe nach Anspruch 1, dadurch gekennzeichnet, daß $R^1$, $R^2$,$R^3$ und $R^4$ jeweils Wasserstoff oder $C_1$-$C_6$-Alkyl, $Q^1$ und $Q^3$ jeweils Wasserstoff, $Q^2$ Isopropyl und $Q^4$ Methyl bedeuten.

**4.** Verdoppelte Azulenquadratsäurefarbstoffe nach Anspruch 1, dadurch gekennzeichnet, daß
$R^1$, $R^3$, $Q^1$ und $Q^3$ jeweils Wasserstoff,
$R^2$ und $Q^2$ jeweils Isopropyl und
$R^4$ und $Q^4$ jeweils Methyl bedeuten.

**5.** Optisches Aufzeichnungsmedium, enthaltend einen Träger sowie einen strahlungsempfindlichen, Farbstoff und gegebenenfalls Bindemittel enthaltenden dünnen Beschichtungsfilm, dadurch gekennzeichnet, daß der Farbstoff die Formel I

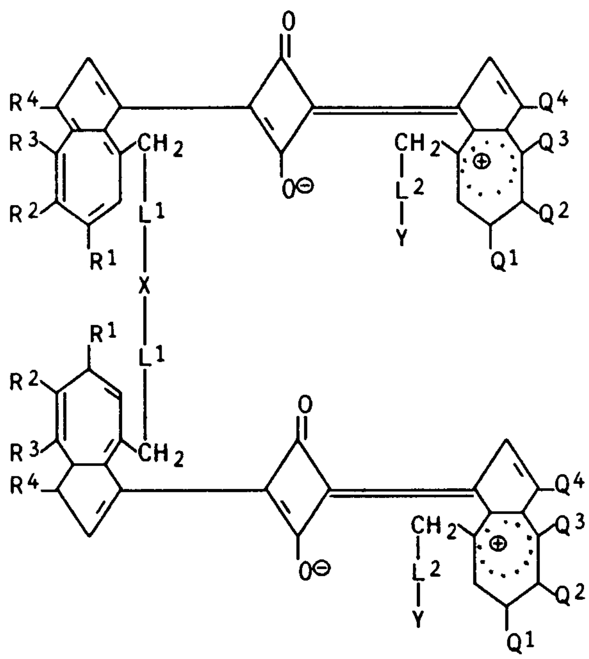

(I)

aufweist, in der

X einen Rest der Formel CO-O-M-O-CO, CO-$NR^5$-M-$NR^5$-CO, O-CO-M-CO-O oder $NR^5$-CO-M-CO-$NR^5$, worin $R^5$ für Wasserstoff, $C_1$-$C_{12}$-Alkyl, $C_2$-$C_{12}$-Alkenyl, $C_5$-$C_7$-Cycloalkyl oder gegebenenfalls substituiertes Phenyl und M für $C_1$-$C_{12}$-Alkylen, Phenylen, Biphenylen oder einen Rest der Formel $(C_2H_4O-)_nC_2H_4$ oder

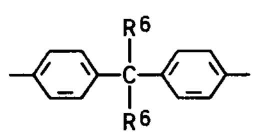

stehen, worin n die Bedeutung von 1 bis 10 und $R^6$ jeweils die Bedeutung von Wasserstoff oder $C_1$-$C_4$-Alkyl besitzen,

Y Wasserstoff, Cyano oder einen Rest der Formel CO-$OR^5$, CO-$NHR^5$, $OR^5$, O-CO-$OR^5$, NH-C0-$R^5$, NH-CO-$OR^5$ oder NH-$SO_2$-$R^5$, worin $R^5$ jeweils die obengenannte Bedeutung besitzt,

$L^1$ und $L^2$ unabhängig voneinander jeweils eine chemische Bindung oder $C_1$-$C_{12}$-Alkylen, das gegebenenfalls durch Phenyl substituiert ist, und

$R^1$, $R^2$, $R^3$, $R^4$, $Q^1$, $Q^2$, $Q^3$ und $Q^4$ unabhängig voneinander jeweils Wasserstoff oder $C_1$-$C_{12}$-Alkyl, das durch Halogen, $C_1$-$C_{12}$-Alkoxy, gegebenenfalls substituiertes Phenyl, $C_1$-$C_{12}$-Alkoxycarbonyl oder Cyano substituiert sein kann,

bedeuten, mit der Maßgabe, daß wenn $R^4$ und/oder $Q^4$ Wasserstoff bedeuten, an den Azulenringen die Ringpositionen der Substituenten $CH_2$-$L^1$-X-$L^1$-$CH_2$ und $R^3$ und/oder $CH_2$-$L^2$-Y und $Q^3$ auch gegeneinander vertauscht sein können.

**6.** Optisches Aufzeichnungsmedium nach Anspruch 5, dadurch gekennzeichnet, daß $R^1$, $R^2$, $R^3$, $R^4$, $Q^1$, $Q^2$, $Q^3$ und $Q^4$ jeweils Wasserstoff oder $C_1$-$C_6$-Alkyl bedeuten.

**7.** Optisches Aufzeichnungsmedium nach Anspruch 5, dadurch gekennzeichnet, daß $R^1$, $R^2$, $R^3$ und $R^4$ jeweils Wasserstoff oder $C_1$-$C_6$-Alkyl, $Q^1$ und $Q^3$ jeweils Wasserstoff, $Q^2$ Isopropyl und $Q^4$ Methyl bedeuten.

8. Optisches Aufzeichnungsmedium gemäß Anspruch 5, dadurch gekennzeichnet, daß $R^1$, $R^3$, $Q^1$ und $Q^3$ jeweils Wasserstoff, $R^2$ und $Q^2$ jeweils Isopropyl und $R^4$ und $Q^4$ jeweils Methyl bedeuten.

9. Verdoppelte Azulenderivate der Formel II

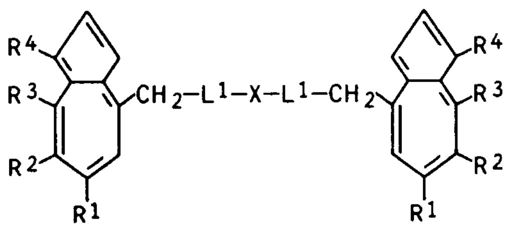

(II),

in der

X einen Rest der Formel CO-O-M-O-CO, CO-$NR^5$-M-$NR^5$-CO, O-CO-M-CO-O oder $NR^5$-CO-M-CO-$NR^5$, worin $R^5$ für Wasserstoff, $C_1$-$C_{12}$-Alkyl, $C_2$-$C_{12}$-Alkenyl, $C_5$-$C_7$-Cycloalkyl oder gegebenenfalls substituiertes Phenyl und M für $C_1$-$C_{12}$-Alkylen, Phenylen, Biphenylen oder einen Rest der Formel $(C_2H_4O-)_nC_2H_4$ oder

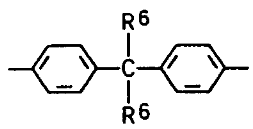

stehen, worin n die Bedeutung von 1 bis 10 und $R^6$ jeweils die Bedeutung von Wasserstoff oder $C_1$-$C_4$-Alkyl besitzen,

$L^1$ eine chemische Bindung oder $C_1$-$C_{12}$-Alkylen, das gegebenenfalls durch Phenyl substituiert ist, und

$R^1$, $R^2$, $R^3$ und $R^4$ unabhängig voneinander jeweils Wasserstoff oder $C_1$-$C_{12}$-Alkyl, das durch Halogen, $C_1$-$C_{12}$-Alkoxy, gegebenenfalls substituiertes Phenyl, $C_1$-$C_{12}$-Alkoxycarbonyl oder Cyano substituiert sein kann, bedeuten.

Europäisches Patentamt

# EUROPÄISCHER RECHERCHENBERICHT

Nummer der Anmeldung

EP 91 12 0867

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| D,A | EP-A-0 310 080 (BASF)<br>* Zusammenfassung; Beispiele *<br>--- | 1-9 | C09B57/00<br>G11B7/24<br>C07C69/608<br>C07C69/007<br>C07C69/76<br>C07C233/05<br>C07C233/65<br>C07C233/10 |
| D,A | EP-A-0 341 541 (BASF)<br>* Zusammenfassung; Beispiele *<br>--- | 1-9 | |
| A | EP-A-0 295 144 (CANON)<br>* Zusammenfassung *<br>* Seite 12, Zeile 5 - Seite 14, Zeile 30 *<br>----- | 1-9 | |
| | | | **RECHERCHIERTE SACHGEBIETE (Int. Cl.5 )** |
| | | | C09B<br>G11B<br>C07C |

Der vorliegende Recherchenbericht wurde für alle Patentansprüche erstellt

| Recherchenort | Abschlußdatum der Recherche | Prüfer |
|---|---|---|
| DEN HAAG | 15 APRIL 1992 | DAUKSCH H.J. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)